# EUROPEAN PATENT SPECIFICATION

(11) **EP 1 407 677 B1**
(45) Date of publication and mention of the grant of the patent: **31.08.2005**
(21) Application number: 03027512.7
(22) Date of filing: 03.05.2002
(51) Int. Cl.: A23L 1/0562, A23L 1/305, A23J 3/34, A23J 3/06, A61K 38/01, A23L 1/302, A23L 1/303, C07K 14/46, A61P 19/10

(54) **Compositions comprising partly-hydrolized fish gelatin and use thereof**
Teilhydrolysierte Fischgelatine enthaltende Zubereitungen und deren Verwendung
Compositions contenant de la gélatine de poisson partiellement hydrolysée et leur utilisation

(30) Priority: 02.07.2001 IT RM20010380
(43) Date of publication of application: 14.04.2004
(62) Divisional of application: 02425280.1
(73) Proprietor: BIOPROGRESS S.p.A., I-00165 Rome (IT)
(72) Inventor: Bonanomi, Michele, 00198 Roma (IT); De Gregorio, Mauro, 00179 Roma (IT)
(74) Representative: Tansini, Elio Fabrizio

(56) References cited:
- WO-A-00/10525
- WO-A-00/61117
- DATABASE WPI Section Ch, Week 199515 Derwent Publications Ltd., London, GB; Class B03, AN 1995-110552 XP002269652 & JP 07 033668 A (MARUHA CORP), 3 February 1995 (1995-02-03)
- DATABASE WPI Section Ch, Week 200002 Derwent Publications Ltd., London, GB; Class D13, AN 2000-016462 XP002218542 & JP 11 266803 A (ISHIZUKA Y), 5 October 1999 (1999-10-05)

## Description

The present invention relates to use of a partly-hydrolysed fish gelatin as a medical speciality. In particular, the present invention relates to use of partly-hydrolysed fish gelatin for use as supplements in the medical field, as a source of easily assimilable amino acids useful for trophism of the osteo-articular system due to a deficient supply, an increased consumption or a defective absorption of amino acids, both in the human and animal field.

JP 7033668 discloses (cf. WPI abstract 1995-110552) a composition for treating osteoporosis comprising shark cartilage and fish bone powder.

It is known, that gelatin obtained from connectivum contains essential and nonessential amino acids in proportions corresponding to those of the bone, the articulations, the keratin and other connective tissues. Oral administration of same to men and laboratory animals, in amounts at least as high as 100 mg/kg, showed a favourable effect under various situations and conditions. By way of example, oral administration of same to an amount of 10 g/day has a well-documented effect on pain and motility in patients suffering from arthrosis, osteoporosis and other diseases of the locomotor apparatus (Adam M., Therapiewoche (1991) 41 2456-61).

In an amount of 100 mg/kg/day it improves hair trophism both in men and laboratory animals (Silvestrini B., "Method for increasing hair growth" US Patent 4,749,684 (1988); Scala J., Hollies N.R.S., Sucher K.P., Nutr. Report Inter. (1976) 13 579-592).

Oral administration of gelatin to the above mentioned dosages is not easy and it is often badly tolerated because this substance in the stomach swells giving rise to an unpleasant feeling of repletion. In addition, in some persons it may cause reaction of the allergic type.

To avoid the above mentioned drawbacks, resorting to a completely hydrolysed gelatin improving amino acid absorption was proposed in the past, so as to enable dosage halving and avoid gastric trouble (Silvestrini B., and Kirschner G., "Integratori alimentari e dietetici contenenti gelatina idrolizzata e loro uso in campo medico" Patent IT 01299131).

However, the completely hydrolysed gelatin too has some drawbacks. In patent IT 01299131 the gelatin hydrolysis is obtained by chemical route or, alternatively, by enzymatic route.

It is known that gelatin hydrolysis obtained by chemical route, is generally carried out by heating with concentrated hydrochloric acid to a temperature of about 110°C for a time included between 18 and 24 hours. This operating modality appears of difficult application on a preparation scale. In addition, recent bibliographic references (Fountoulakis M., Lahm H.W., J. Chromatogr. A, (1998) 826 109-134) clearly highlight formation of decomposition products the nature of which is a function of the employed experimental conditions.

It is known that gelatin hydrolysis, obtained by enzymatic route is surely a less drastic process, but it has drawbacks as well.

A first drawback is connected with the presence of free sulfurised amino acids derived from hydrolytic processes. Owing to the presence of sulfurised amino acids the organoleptic properties of the gelatin are worsened, due to sulphur smell and aftertaste that cannot be accepted by all consumers and patients.

Alternatively, mixtures could be used that consist of the individual amino acids in the proportions found in collagen.

The last-mentioned solution too is not devoid of shortcomings. A first shortcoming refers to the related high costs for accomplishment of these amino acid mixtures. A high cost is hardly compatible with the features of a treatment involving administrations over long periods of time, in relatively high amounts.

In addition, the presence of potentially toxic impurities is not to be excluded, which impurities result from both extractive and synthetic processes employed for obtaining the individual amino acids constituting the mixture.

The technical problem to be solved consists in making a source of amino acids more bioavailable for the metabolism of the human and animal organism, said amino acids being easily assimilable and coming from a gelatin that does not show the drawbacks of the known art.

In particular, the technical problem to be solved consists in making a source of amino acids less bioavailable for the metabolism of the human and animal organism, so that diseases arising from a lack of amino acids in the living organism can be treated while avoiding the drawbacks of the known art.

The solution to this problem is proposed by the Applicant that found it useful to employ a partly-hydrolysed fish gelatin.

It is first object of the present invention to provide a composition comprising a partly-hydrolysed fish gelatin for use as a medical speciality, having the features set out in the appended independent claim.

It is a further object of the present invention to provide use of a composition comprising a partly-hydrolysed fish gelatin for preparation of a medical speciality having the features set out in the appended independent claim.

Other preferred embodiments of the present invention are described in the appended sub-claims. Further technical features and the advantages of the invention will be best understood from the following detailed description.

The partly-hydrolysed gelatin being the object of the present invention is conceived as an available source of amino acids easily assimilable by the organism. Preferably, the natural fish gelatin is submitted to a hydrolysis treatment. Hydrolysis can be carried out by chemical route or, alternately, by enzymatic route following methods that are known to those skilled in the art. The true hydrolysis is carried out until a partly-hydrolysed gelatin is obtained in which the macromolecules have a molecular weight not exceeding 50,000 Daltons. The degree of partial hydrolysis is obtained in such a manner that the native fish gelatin, following the hydrolysis process, loses its native original structure. The native structure is responsible for gelatin gelation. Hydrolysis causes breaking of the interchain bonds of the native gelatin and does not involve formation of free sulfurised amino acids. Treatment to which collagen (in this case fish collagen) is submitted brings to achievement of a gelatin form which is cold-soluble in water, which has lost its gel-forming feature. The product is defined as a non-gelling water-soluble gelatin. The particular chemical structure of partly-hydrolysed gelatin which is the object of the present invention allows the gelatin to be easily assimilable by the organism and, consequently allows use of lower dosages than those required if native gelatin were used. In addition, since the partly-hydrolysed gelatin being the object of the invention does not contain free sulfurised amino acids, it has no disagreeable aftertaste which is typical of the last-mentioned products. Finally, presently there are not in the literature announcements of a possible allergenic power which was on the contrary practically found and mentioned in the literature with reference to native gelatin.

The partly-hydrolysed gelatin which is the object of the present invention achieves two important advantages with respect to both the fully hydrolysed gelatin and the reconstituted gelatin obtained by mixing the constituent amino acids thereof in their original proportion:
- it improves acceptability of treatment by a patient avoiding the disagreeable aftertaste due to free sulfurised amino acids;
- it greatly reduces the treatment cost.

As compared with native gelatin, the partly-hydrolysed gelatin on the contrary has the following advantages:
- it improves treatment acceptability by the patient avoiding the feeling of gastric swelling due to gelation;
- it reduces the required doses for obtaining the desired effects.

This result could be expected in the light of the prior art that led to think that a full hydrolysis of the native gelatin was necessary and essential for improving absorption of the gelatin-constituent amino acids and reduce active dosages thereof.

The partly-hydrolysed gelatin being the object of the present invention applies to all gelatin uses that are not linked to its original physico-chemical properties, such as swelling in the presence of water, but that are linked to its ability to supply the necessary amino acids in a readily absorbable form where there is a lack of amino acids or under unhealthy conditions that can take advantage of an additional supply of amino acids.

In an embodiment being the object of the invention the partly-hydrolysed fish gelatin is provided for use as a medical speciality.

Another embodiment being the object of the invention provides a composition comprising a partly-hydrolysed fish gelatin for use as a medical speciality.

The compositions comprising partly-hydrolysed gelatin efficaciously apply in the medical field and in the veterinary field. In the veterinary field, the compositions comprising partly-hydrolysed gelatin can be used with reference to pets such as dogs and cats for example.
The partly-hydrolysed fish gelatin is used for preventive and/or curative treatment of diseases associated with a lack of amino acids in the living organism. In particular, for the preventive treatment of, and/or as an adjuvant in osteoporosis.

Preferably, the composition of the medical speciality is in a form adapted for oral administration.

In addition, the compositions being the object of the present invention can be employed as adjuvants in therapies where use of other specific drugs is provided.

Advantageously, the composition of the medical speciality is in the form of a ready-dissolution granular powder, masticable tablets or effervescent tablets. In addition, water-based sterile solutions comprising the compositions of the present invention already in a dissolved form ready for use can be prepared.

Advantageously, the partly-hydrolysed fish gelatin is in combination with calcium carbonate and vitamin D3 (cholecalciferol).

Advantageously, the composition of the medical speciality efficiently applies under unhealthy conditions, such as senescence, convalescence, pregnancy, and chronic renal insufficiency and in sports activities.

The compositions of the invention efficiently apply for the above mentioned cases as an alternative to other amino acid sources, such as meat that can be assimilated less easily or can involve taking of useless or dangerous components.

In addition, they can be employed in association with low-calory diets in treating obesity in order to avoid the risk of an insufficient amino acid supply.

The compositions of the invention can be administered to patients through true treatment cycles.

For instance, a treatment cycle involves administration of an amount of partly-hydrolysed fish gelatin included from 4 to 10 g/day. Preferably, in an amount of 5-6 g/day.

For instance, administration takes place twice a day at the main meals for a period of time of 8 to 16 weeks. Preferably for a period of time in the range of 10 to 12 weeks. In addition, provision is made for a method involving preventive and/or curative treatment of diseases sharing a deficient supply or an increased consumption of amino acids, or a defect in the amino acid absorption in the organism comprising at least one treatment cycle during which a composition comprising partly-hydrolysed fish gelatin as claimed is administered.

### Oral absorption of the amino acids contained in the gelatin

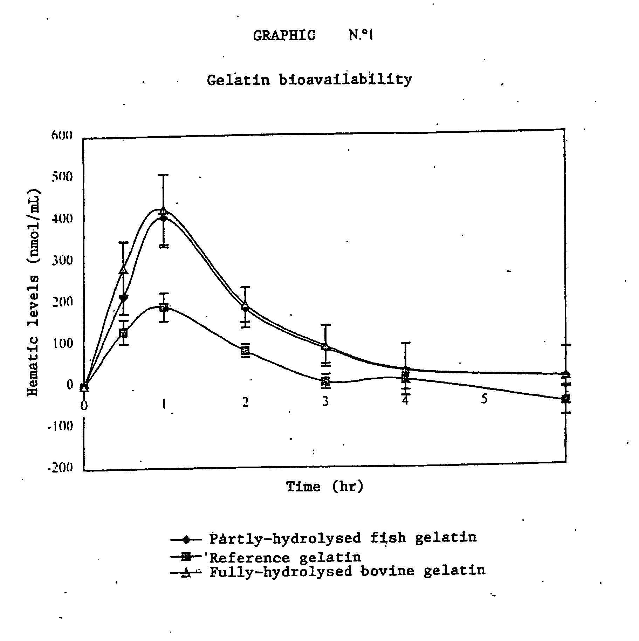

To two parallel groups of eight rabbits on an empty stomach 100 mg/kg of partly-hydrolysed (non gelling) fish gelatin with an average molecular weight not exceeding 50,000 Dalton and respectively 100 mg/kg of reference gelatin of bovine origin, non hydrolysed and with marked gelling properties at room temperature were administered
through a gastric tube. At predetermined times samples of blood from the central artery of the ear were carried out to determine the plasma concentration of the free amino acids. The analysis was conducted after precipitation of the plasma proteins, lyophilizing of the residue, transformation into a derivative by phenyl isothiocyanate and HPLC analysis Pico Tag®. The quantitative determination was carried out on six amino acids present in greater quantities in the gelatin and more particularly: glycine, alanine, arginine, proline, hydroxyproline and glutamic acid. Data reproduced in Table 1 and Figure 1 are expressed in nmoles/ml as the sum of the six amino acids analysed after subtraction of basal values. From data in Table 1 and in the graph in Fig. 1, it is possible to infer that absorption of partly-hydrolysed fish gelatin, evaluated in terms of free amino acids present in the blood, is about twice with respect to the reference gelatin. The increase is almost superimposable on that previously reproduced with the fully hydrolysed gelatin. The partly-hydrolysed gelatin can be used as a base for preparations adapted for treatment of different pathologic conditions.

In addition the partly-hydrolysed gelatin can be administered in formulations of different form such as ready-dissolution granular powder, masticable pills, tablets soluble in mouth, effervescent tablets, masticable tablets and kit. By way of non-limiting example of the object of the present invention, some of the object of the present invention, some examples of compositions comprising partly-hydrolysed fish gelatin are reproduced hereinafter. The compositions of the following examples were prepared following knowledge and techniques known to a person skilled in the art.

### examples 3-7 out of scope of the invention!

Example 1: ready-dissolution granular powder to be used in osteoporosis (1 packet of granular powder contains):

| | | |
|---|---|---|
| Partly-hydrolysed fish gelatin Calcium carbonate (with addition of | g | 2.50 |
| maltodextrin) | g | 1.10 |
| Vitamin D3 (cholecalciferol) | µg | 2.50 |
| Sodium benzosulfimide | mg | 50 |
| Strawberry/vanilla flavour | mg | 114 |
| Citric acid | g | 1.76 |
| Mannitol | mg | 250 |
| 50% dimethicone powder | mg | 60 |
| Red colour | mg | 2 |

### Example 2: tablet

| | | |
|---|---|---|
| Partly-hydrolysed fish gelatin Calcium carbonate (with addition of | g | 2.50 |
| maltodextrin) | g | 1.10 |
| Vitamin D3 (cholecalciferol) | µg | 2.50 |
| Sodium benzosulfimide | mg | 50 |
| Strawberry/vanilla flavour | mg | 117 |
| Citric acid | g | 1.50 |
| Mannitol | mg | 250 |
| 50% dimethicone powder | mg | 60 |
| PEG 6000 | mg | 60 |
| Red colour | mg | 2 |

Example 3: ready-dissolution granular powder to be used in convalescence (1 packet of granular powder contain):

| | | |
|---|---|---|
| Partly-hydrolysed fish gelatin | g | 2.50 |
| Lysine hydrochloride | mg | 150 |
| Taurine | mg | 50 |
| Vitamin B6 | mg | 1 |
| Vitamin C | mg | 45 |
| Sodium benzosulfimide | mg | 50 |
| Sodium cyclamate | mg | 50 |
| Banana flavour | mg | 154 |

Example 4: ready-dissolution granular powder to be used in senescence (1 packet of granular powder contains):

| | | |
|---|---|---|
| Partly-hydrolysed fish gelatin | g | 2.50 |
| EPA- and DHA-rich sea fish oil | g | 2 |
| Vitamin E | mg | 10 |
| Vitamin C | mg | 45 |
| Betacarotene | mg | 5 |
| Sodium benzosulfimide | mg | 50 |
| Sodium cyclamate | mg | 50 |
| Vanilla flavour | mg | 150 |

Example 5: ready-dissolution granular powder to be used in pregnancy and/or nursing (1 packet of granular powder contains):

| | | |
|---|---|---|
| Partly-hydrolysed fish gelatin | g | 2.50 |
| Lysine hydrochloride Iron gluconate (corresponding to | mg | 150 |
| mg 7 iron) | mg | 60 |
| Calcium glycerophosphate | g | 1 |
| Vitamin A | mg | 0.4 |
| Vitamin B1 | mg | 0.5 |
| Vitamin B2 | mg | 0.5 |
| Vitamin B3 | mg | 7.5 |
| Vitamin B6 | mg | 1 |
| Vitamin C | mg | 25 |
| Vitamin D3 (cholecalciferol) | µg | 2.5 |
| Vitamin H | mg | 0.07 |
| Sodium benzosulfimide | mg | 50 |
| Vanilla flavour | mg | 150 |

Example 6: ready-dissolution granular powder to be sed in micro circulation and vein troubles (1 packet of granular powder contains):

| | | |
|---|---|---|
| Partly-hydrolysed fish gelatin | g | 2.50 |
| Centella Asiatica (triterpene fraction) | mg | 15 |
| Vitamin C | mg | 45 |
| Vitamin E | mg | 7 |
| Sodium benzosulfimide | mg | 50 |
| Sodium cyclamate | mg | 50 |
| Strawberry flavour | mg | 140 |

Example 7: ready-dissolution granular powder to be used in association with low-calory diets (1 packet of granular powder contains):

| | | |
|---|---|---|
| Partly-hydrolysed fish gelatin | g | 2.50 |
| Sodium benzosulfimide | mg | 50 |
| Strawberry flavour | mg | 140 |

Example 8: ready-dissolution granular powder to be used as medical speciality in osteoporosis (1 packet of granular powder contains):

| | | |
|---|---|---|
| Partly-hydrolysed fish gelatin Calcium carbonate (with addition of | g | 2.50 |
| maltodextrin) | g | 1.25 |
| Vitamin D3 (cholecalciferol) | µg | 4.4 |
| Sodium benzosulfimide | mg | 50 |
| Strawberry/vanilla flavour | mg | 114 |
| Citric acid | g | 2.175 |
| Mannitol | mg | 250 |
| 50% dimethicone powder | mg | 60 |
| Red colour | mg | 2 |

**Table 1**

| Partly-hydrolysed fish gelatin | | | Reference gelatin | | | Fully-hydrolysed bovine gelatin | | |
|---|---|---|---|---|---|---|---|---|
| Time (hr) | nmol /mL | Standard error | Time (hr) | nmol /mL | Standard error | Time (hr) | nmol/ mL | Stand ard error |
| 0 | 0 | 0 | 0 | 0 | 0 | 0 | 0 | 0 |
| 0.5 | 216.01 | 41.74 | 0.5 | 131.50 | 28.43 | 0.5 | 285.10 | 64.22 |
| 1 | 405.75 | 70.44 | 1 | 190.13 | 34.39 | 1 | 424.7 4 | 84.16 |
| 2 | 183.13 | 45.51 | 2 | 81.38 | 16.69 | 2 | 193.2 1 | 41.89 |
| 3 | 85.88 | 36.21 | 3 | 5.25 | 17.74 | 3 | 91.40 | 49.58 |
| 4 | 31.50 | 30.45 | 4 | 10.50 | 25.29 | 4 | 33.84 | 62.81 |
| 6 | 10.63 | 22.86 | 6 | - 49.75 | 32.85 | 6 | 11.98 | 70.18 |

## Claims

1. Use of a partly-hydrolysed fish gelatin, wherein said partly-hydrolysed gelatin has a molecular weight not exceeding 50,000 Daltons, to prepare a medical speciality for the treatment of osteoporosis.

2. Use according to claim 1, in which the partly-hydrolysed fish gelatin is in association with an effervescent system which can release nascent calcium in contact with a water-based solution.

3. Use according to claim 2, in which the effervescent system which can release nascent calcium comprises calcium carbonate and an acid, preferably citric acid.

4. Use according to claim 3, in which the partly-hydrolysed fish gelatin is in association with an effervescent system and vitamin D3.

## Patentansprüche

1. Verwendung einer teilhydrolysierten Fischgelatine, wobei die teilhydrolysierte Gelatine ein Molekulargewicht nicht über 50.000 Dalton aufweist, zur Herstellung eines Arzneimittels für die Behandlung von Osteoporose.

2. Verwendung nach Anspruch 1, wobei die teilhydrolysierte Fischgelatine in Kombination mit einem Brausesystem ist, das in Kontakt mit einer wässrigen Lösung entstehendes Kalzium freigeben kann.

3. Verwendung nach Anspruch 2, wobei das Brausesystem, das entstehendes Kalzium freigeben kann, Kalziumkarbonat und eine Säure enthält, bevorzugt Zitronensäure.

4. Verwendung nach Anspruch 3, wobei die teilhydrolysierte Fischgelatine in Kombination mit einem Brausesystem und Vitamin D₃ ist.

## Revendications

1. Utilisation d'une gélatine de poisson partiellement hydrolysée, où ladite gélatine partiellement hydrolysée a un poids moléculaire non supérieur à 50.000 Dalton, pour la préparation d'un médicament pour le traitement de l'ostéoporose.

2. Utilisation selon la revendication 1, où la gélatine de poisson partiellement hydrolysée est associée à un system effervescent étant à même de délivrer calcium naissant en contact avec un solution à base d'eau.

3. Utilisation selon la revendication 2, où le système effervescent étant à même de délivrer calcium naissant comprend carbonate de calcium et un acide, de préférence acide citrique.

4. Utilisation selon la revendication 3, où la gélatine de poisson partiellement hydrolysée est associée avec un système effervescent et vitamine D₃.
